# EUROPEAN PATENT SPECIFICATION

(11) **EP 1 868 566 B1**
(45) Date of publication and mention of the grant of the patent: **20.03.2013**
(21) Application number: 06726727.8
(22) Date of filing: 10.04.2006
(51) Int. Cl.: A61K 9/00, A61M 31/00

(54) **CONTROLLED RELEASE DEVICES AND STRUCTURAL ELEMENTS FOR USE IN THEIR MANUFACTURE**
VORRICHTUNG ZUR KONTROLLIERTEN ABGABE SOWIE STRUKTURELLE ELEMENTE ZUR VERWENDUNG IN DEREN HERSTELLUNG
DISPOSITIFS D'ADMINISTRATION LENTE ET ELEMENTS STRUCTURELS DESTINES A LA FABRICATION DE CES DISPOSITIFS

(30) Priority: 12.04.2005 GB 0507383
(43) Date of publication of application: 26.12.2007
(73) Proprietor: CASTEX PRODUCTS LIMITED, High Peak, Derbyshire SK22 3HF (GB)
(72) Inventor: WHITEHEAD, Derek James, Stockport, Cheshire SK12 1LJ (GB); WHITEHEAD, Martin John, Macclesfield, Cheshire SK11 0ND (GB)
(74) Representative: Clegg, Richard Ian
(86) International application number: PCT/GB2006/001327
(87) International publication number: WO 2006/109053

(56) References cited:
- EP-A- 0 164 927
- EP-A- 0 243 111
- WO-A-96/01619

## Description

The invention relates to structural elements suitable for use in the manufacture of controlled release devices, especially controlled release boli, and to such release devices for administration of active agents to ruminant animals. Such intraruminal boli are commonly employed to deliver active agents to ruminant animals over extended periods of time.

The invention also relates to the use, in the manufacture of controlled release boli, of such structural elements, which are capable of retaining their integrity so as to carry the active agent during the controlled release.

As may be seen from the following examples, current bolus designs are complex, leading to high component production and assembly costs.

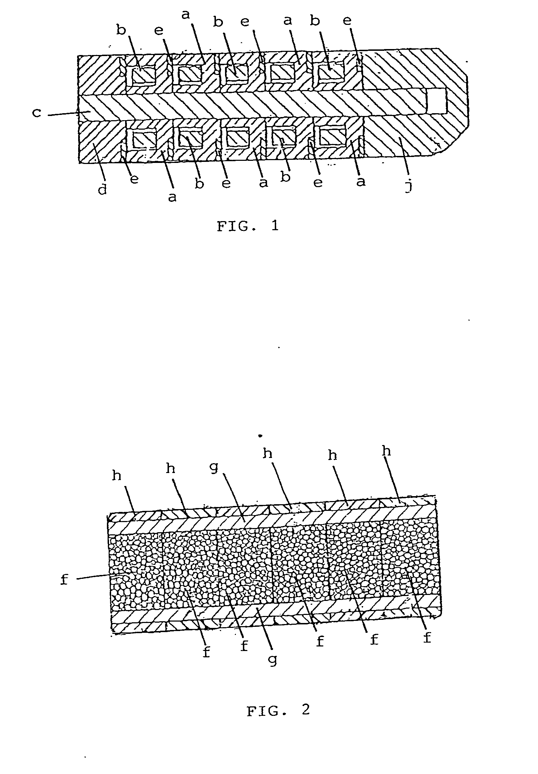

Thus, EP-A-0164927 discloses a pulse release bolus for cattle which has gained wide acceptance and is based on the galvanic corrosion of magnesium as a timing mechanism. A longitudinal section of such a device is illustrated in Figure 1. The device consists of a series of segments [a] of plastics material containing anthelmintic oxfendazole tablets [b] carried on a magnesium alloy support rod [c]. The bolus is weighted by a dense iron mass [j] to avoid regurgitation by the animal. A blank segment [d] provides an initial delay after administration and before an active tablet is released. The junctions between adjacent segments and the weight are sealed with rubber washers [e]. Typically, the device has a length of 90mm and a diameter of 25mm. The device operates by the progressive shortening of the magnesium support rod in the rumen fluid which results in the periodic discard of segments and release of active tablets. The bolus contains 19 separate components of 6 types made from 5 different constructional materials.

EP-A-0243111 discloses a continuous release bolus for cattle, also based on the corrosion of magnesium as a timing mechanism and widely used. A longitudinal section of such a device in accordance with EP-A-0243111 is illustrated in Figure 2. The device consists of a series of tablets [f] inserted into a magnesium alloy support tube [g] the surface of which, apart from the extreme ends, is protected from attack by the rumen contents by close fitting, interlocking rings [h] of plastics material. Typically, the device has a length of 80mm and a diameter of 28mm. The tablets contain the active agent fenbendazole as an anthelmintic, graphite to provide a cathode to the magnesium and disseminated iron particles which serve to weight the bolus, together with binders and soluble agents. The bolus support tube corrodes from both ends, continuously releasing fenbendazole from the erodable tablets [f]. The bolus contains 21 separate components of 6 types made from 3 different constructional materials all produced to close dimensional tolerances.

Smaller devices similar to the above examples may be constructed for sheep.

With reference to EP-A-0243111 (supra), it is to be noted that, as illustrated by this document, iron and graphite combinations have been utilised in bolus manufacture for several years. However, in the manufacture of such degradable (erodable) parts of such boli, the graphite powder, in combination with iron shot particles, sugar and anthelmintic, is used to form a degradable tablet which is cathodic to an enclosing magnesium shell. The function of the graphite is to make the tablet electrically conductive and provide the electrode to promote galvanic corrosion of the magnesium shell. The rumen contents serve as the electrolyte. The iron is present in the form of coarse particles and acts solely as a weighting agent to avoid regurgitation of the bolus by the animal and does not contribute to the strength and integrity of the tablets or bolus. Other degradable tablets containing iron and graphite and used as the central core in bolus construction are described in EP-A-0482947 and WO-A-98/56347.

As will be explained more fully below with reference to the invention, such use as described above of iron and graphite in an erodable composition is in stark contrast with the use of these materials in producing a structural element carrying active and degradable components.

WO-A-96/01619 discloses a bolus for delivery of pulsed doses of biologically active material to a ruminant animal which comprises a series of distinct and separate structural elements, housed within an outer tube of an easily degradable material such as paper, and each defining a respective segment of the bolus. Each element is in the form of a short solid cylinder having a central recess in an axial end thereof, which recessed cylinder defines a dense cathodic sheath for a tablet of active material covered by a plug of magnesium each within the recess. Degradation of the outer tube releases the individual segments, which disperse within the rumen. Galvanic corrosion of the magnesium plug under the influence of the cathodic sheath then releases the tablet. The cathodic sheath may be formed by compacting a 50 - 50 mixture (by volume) of pure iron and graphite powders, thus providing an iron/graphite mixture containing about 13wt% graphite, by weight of the mixture. However, as described there, compacting requires a load as high as 10 tonnes/cm². Moreover, even then, physical properties such as rupture load and water repellancy are not satisfactory.

In the case of cattle with internal parasites the live weight gain of the animal due to the use of boli can more than compensate for the cost of treatment and there is a strong economic case for their use. This may apply particularly to some regions of the Western Hemisphere where the value of the animals is higher than elsewhere. However for sheep the cost saving may be less attractive due to the lower value of the animal. Boli which give extended protection against parasites e.g. for periods which may exceed four months by pulse or continuous release of the active agent are, as illustrated above, generally complex assemblies of several different types of component. Although lower doses of active agent are required for sheep and the boli are smaller and use less materials than those for cattle the component production and assembly costs are much the same for both types of bolus and any savings are generally inadequate to compensate for the lower value of the animal. There is hence a need for less complex and lower production cost devices and this is particularly the case for sheep. Again, there is little doubt that intraruminal anthelmintic boli would find increased use in Southern Hemisphere countries, where animal values are lower, if cheaper variants were available.

The present invention seeks to address the drawbacks and limitations of conventional release devices discussed above.

We have now found surprisingly that, if a bolus has a structural unit compacted from a mixture of iron, graphite and copper but with lower proportions of graphite in the mixture than that of the release devices disclosed in WO-A-96/01619, then both improved performance and ease of manufacture can be achieved.

Moreover, the use of such a structural unit in the manufacture of a release device allows the production of a preferred release device in accordance with the invention in which segments of the bolus are released one at a time for progressive sustained release of active material from respective segments.

According to a first aspect, the present invention provides a structural element suitable for use in the manufacture of a release device for intraruminal administration of an active agent to a ruminant animal, which structural element is of a compacted material, which material comprises a mixture of powdered iron, graphite and copper, the graphite being present in the mixture in an amount of from 2 to 7wt%, the copper in an amount of from 0 to 5wt% and the iron in an amount of from 88 to 98wt%, by weight of the total amount of iron, copper and graphite.

The structural element is particularly suitable for use as a segment of a structural unit comprising an assembly thereof.

The structural element may have a radial cross section which is circular and, in particular, may have a cylindrical configuration especially suitable for a cylindrical bolus.

The structural element preferably comprises a cylindrical element having a central cylindrical aperture therein, coaxial with the structural element and adapted to receive, with an interference fit, a core of anodic material.

At least one end face of the structural element may have a recess therein adapted to receive a dosage form of an active agent composition. Indeed, at least one end face may have a plurality of recesses, typically from 2 to 10, each adapted to receive a respective said dosage form. For example, a plurality of circular section recesses may accommodate a plurality of circular section tablets.

The recess or recesses may be of any desired shape in order to accommodate any desired shape of dosage form and may be of any volume sufficient to accommodate a pulsed dose of active agent. For example, a circular section recess is suitable for accommodating a correspondingly shaped tablet in the form of a disc while an annular recess is suitable for receiving a ring shaped tablet. The recess may be located in any position in the end face but is preferably spaced from each of the internal and external peripheries of the structural element. However, a ring shaped tablet may be accommodated within a circular section recess coaxial and radially coextensive with an aperture adapted to receive a core of anodic material as described above. However, the inner diameter of the ring shaped tablet must then be sufficiently wide to avoid significant contact with the core during assembly of the bolus. As described below, when one or more recesses are provided on each of opposed faces, recesses in adjacent faces of adjacent structural elements may cooperate to hold a single dosage form.

A plurality of structural elements as described above may be assembled to provide a structural unit, preferably of cylindrical form.

Such a structural element may have at least one recess in each of opposed end faces thereof, whereby, in the assembly of structural elements, the or each recess in an end face of a structural element being in register with a corresponding recess in an adjacent end face of an adjacent structural element, respective corresponding recesses thereby cooperating with one another to house a single dosage form.

A structural element in accordance with the first aspect of the invention may be prepared by compacting, by, for example, pressing or moulding, the material comprising the mixture of powdered iron, graphite and copper.

The powders to be compacted preferably have a mesh size, as measured by British Standard (BS) 410, within the range 100 - 400 mesh, more preferably 300 mesh.

The compaction of the material comprising powdered graphite, iron and any copper may result in a release device having a density of 3 - 6.5 gm/ml. This density depends upon the pressure applied and the proportional amount of graphite and copper present.

In a preferred embodiment, the material of the structural element consists of a mixture of iron, in an amount of from 88 to 98wt%, copper, in an amount of 0 to 5wt% and graphite, in an amount of 2 to 7wt%, by weight of total material.

In a more preferred embodiment, the amount of copper in the structural element is at least 1wt%, still more preferably from 1 to 4wt%, especially about 3wt%. The addition of copper may provide a harder compacted material and may reduce the tonnage required for compaction.

The structural element material may comprise a mixture which additionally includes a lubricant, for example, magnesium stearate. If present, the amount is then preferably up to 2wt%, more preferably up to 1wt%, based on the total weight of the material.

With reference to the proportional amount of graphite, if the amount is too low, adequate lubricity and freedom from moisture penetration of the structural units by the rumen contents may not be guaranteed. If the amount is too high, the structural unit will become too fragile.

With respect to the manufacture, and ultimate performance in a release device, of a structural element embodying the invention, the iron, graphite and copper have the following influences.

### Iron

(a) Strength - aids assembly, prevents failure.
(b) Density - sufficiently high to avoid regurgitation.
(c) Electrode Potential - enhances corrosion.
(d) Mouldability - can be pressure formed from powder.
(e) Cost - it is inexpensive.

### Graphite

(a) Processing - enhances mouldability.
(b) Lubrication - no additional lubricant normally needed for moulding.
(c) Porosity - heals porosity so that the segments do not absorb water.
(d) Water repellant - very high so that the water does not wet and penetrate the segments.
(e) Electrical conductivity - provides sufficient electrical conductivity or, in any event, does not reduce the conductivity of iron and copper.
(f) Assembly - improves physical properties, especially shear strength.

### Copper

(a) Strength - augments strength conferred by iron.
(b) Mouldability - enhanced by copper.
(c) Cost - it is relatively inexpensive.

From the above, it can be seen that, in contrast with the bolus construction of EP-A-0243111, described previously, where the iron serves only as a weighting agent so as to avoid regurgitation by the animal, here it performs simultaneously the functions of a weighting agent and a cathodic element and also provides the required strength. Moreover, copper further augments the strength of the bolus.

Similarly, in contrast with the bolus disclosed in WO-A-96/01619, also comprising structural elements made up of iron and graphite, the structural element in accordance with the invention, which contains substantially lower proportions of graphite (2 - 7wt%) as compared with that of the structural elements of WO-A-96/01619 (about 13wt%), it is possible to compact the material into pressings having the desired physical properties such as rupture load; the physical properties of pressings from the material of WO-A-96/01619 are so inferior to those of a structural element of the invention as to render them unsuitable for fabrication into a bolus in accordance with the invention.

According to a second aspect, the present invention provides a release device for intraruminal administration of an active agent such as a medicament or dietary supplement, to a ruminant animal, the device having a degradable member, a non-degradable structural unit and an active agent composition carried by the non-degradable structural unit, which structural unit is made from a compacted material, which material comprises a mixture of powdered iron, graphite and copper, the graphite being present in the mixture in an amount of from 2 to 7wt%, the copper in an amount of from 0 to 5wt% and the iron in an amount of from 88 to 98wt% of the total weight of iron, copper and graphite, whereby the compacted material is capable of enhancing the density of the release device and capable of promoting galvanic corrosion, by rumen juices, of the degradable member, the degradable member and non-degradable structural unit being arranged so that intraruminal corrosion of the degradable member exposes the non-degradable structural unit and active agent composition carried thereby, thus enabling release of the active agent into the rumen juices.

According to a third aspect, the invention provides the use, in the manufacture of a release device for intraruminal administration of an active agent to a ruminant animal, of a non-degradable structural unit of a compacted material, which material comprises a mixture of powdered iron, graphite and the graphite being present in the mixture in an amount of from 2 to 7wt%, the copper in an amount of from 0 to 5wt% and the iron in an amount of from 88 to 98wt% of the total weight of iron, copper and graphite, whereby the compacted material is capable of enhancing the density enhancing component and a component capable of promoting galvanic corrosion, by rumen juices, of a degradable member of the release device.

In a preferred release device in accordance with a second aspect of the invention, the non-degradable structural unit thereof preferably comprises at least one structural element of the first aspect of the invention and more preferably comprises an assembly of such structural elements used as respective segments of a structural unit.

A release device in accordance with a second aspect of the invention is particularly suitable for the treatment of cattle and sheep with anthelmintics for the control of intestinal parasites which represents a major share of the animal health market for cattle and sheep but it may also be used with other active agents including therapeutics and nutrients. The release device is preferably an intraruminal bolus where the active agent release mechanism is determined by the corrosion of magnesium by the rumen fluid.

Thus, preferably the degradable member comprises magnesium, more preferably, a magnesium alloy. Still more preferably, the degradable member is a magnesium alloy core, which may be for example, rod-shaped or tubular, as known from the prior art.

The magnesium alloy preferably contains at least 70wt% of magnesium and any one or more of aluminium (preferably up to 15wt%), zinc (preferably up to 5%), copper (preferably up to 4wt%), manganese (preferably up to 2wt%), silicon (preferably up to 1.5wt%), and zirconium (preferably up to 0.8wt%).

In a preferred release device embodying the invention, in which a plurality of segments of the non-degradable structural unit surround a degradable core, the structural unit serves several functions simultaneously, namely
(a) it provides a support for dosage forms of the active agent;
(b) it serves to prevent ingress of rumen juices into the release device until degradation of the degradable core;
(c) it confines corrosion of the degradable core to regions not surrounded by the structural unit;
(d) it enhances the density of the release device to prevent regurgitation by the animal; and
(e) it promotes galvanic corrosion of the degradable core.

Thus, as previously mentioned, unlike certain prior art release devices (see, for example, EP-A-0243111, supra) where the iron serves only to add weight to the device, in contrast, in a release device embodying the invention, the iron is also incorporated in a structural unit, which structural unit simultaneously confers strength upon the device, provides a cathode for promoting galvanic corrosion of the anode and provides a carrier for a dosage form of the active agent. This has the advantage that no further components are needed to provide the desired compressive strength and structural integrity, for example an outer layer of plastics material is not needed, sealing washers between respective segments are not needed and the degradable component can be a core, rather than a tube. This simplified approach reduces the complexity of the device and its manufacture, and reduces costs.

Preferably, the release device includes a structural unit comprising a plurality of segments in face to face relation with one another, including segments having, in at least one axial end face thereof, a recess capable of receiving a dosage form of the active agent composition.

Suitably, the segments are sequentially released into the rumen juices as the degradable member degrades. The segments may be interlinked to assist in preventing the ingress of moisture into the device. Alternatively or additionally the segments may simply abut one another within the device.

In a first preferred embodiment, each of a plurality of adjacent segments in face to face relation with one another has one axial end face having at least one recess in face to face relation with an axial end face of an adjacent segment free from a recess.

In an alternative, second preferred embodiment, each of adjacent segments in face to face relation with one another has at least one recess in each axial end face, each recess in an axial end face lying in register with a corresponding recess in an adjacent end face of an adjacent segment, whereby respective adjacent opposed recesses together define a cavity in which sits a dosage form. The dosage form therefore contains twice the dosage of a dosage form in a single recess.

By increasing the length of each segment, this second embodiment allows a greater time interval between albeit double dosages. Thus, such a double dose could be given less frequently for example, at 40-day as opposed to 20-day intervals. In this way, the time taken for certain parasites to become immune to the drug if administered too often can be delayed.

A similar effect may be achieved using structural elements in accordance with the above first embodiment as segments in an assembly thereof in which adjacent segments were arranged so that those respective axial end faces having at least one recess therein lay in face to face relation with one another such that respective adjacent opposed recesses in register with one another together defined a cavity for a double dosage form, while those respective axial end faces free from respective recesses also lay in face to face relation with one another so as to provide a longest length between adjacent faces containing recesses.

Suitably, the non-degradable structural unit is arranged so that it protects a large proportion of the total peripheral surface of the degradable member from the rumen juices. Since the non-degradable structural unit does not degrade in the rumen juices there is no need to provide a separate protective layer of plastics material or resin. Preferably, the degradable member is a core and the non-degradable structural unit is arranged around the core to protect the peripheral surfaces of the core.

Both ends of the core may be uncovered so that they are exposed to the rumen juices when the device is administered. In such a case, the peripheral surface between the ends may be entirely covered by the structural unit. Alternatively, one of the longitudinal ends of the core may also be covered, either by a segment of the structural unit, shaped so as to provide a covering element, or simply by means of a plug.

The structural unit preferably comprises a plurality of annular section segments having respective central apertures together defining a central aperture of the structural unit and the degradable member is a solid cylindrical rod making an interference fit with the central aperture of the structural unit.

The active agent may be provided in an active agent composition. Preferably the active agent composition is in dosage, for example, tablet, form.

A particularly preferred release device in accordance with the second aspect of the invention is a sustained release bolus comprising
a structural unit comprising a plurality of annular segments in face to face relation with one another and having respective central apertures together defining a central cylindrical aperture of the structural unit;
a central cylindrical core of a magnesium alloy making an interference fit within the central cylindrical aperture of the structural unit;
the structural unit including segments having, in at least one axial end face thereof, at least one recess; and
a dosage form of a medicament housed in each respective recess of the segments;
each annular segment comprising a compacted material, which material comprises a mixture of powdered iron, graphite and copper, the graphite being present in the mixture in an amount of from 2 to 7wt%, the copper in an amount of from 0 to 5wt% and the iron in an amount of from 88 to 98wt%, by weight of the total weight of iron, copper and graphite, which compacted material is capable of enhancing the density of the bolus promoting galvanic corrosion of the magnesium alloy core; and
at least one axial end region of the core being exposed to allow the said galvanic corrosion thereof.

In contrast with the construction of release device shown in WO-A-96/01619, which provides for release of the segments for dispersion within the rumen, the above release device embodying the invention provides release of the segments one at a time.

This construction embodying the invention allows more accurate estimation of the time of release of the medicament and avoids the formation of an unwanted oxide layer between the magnesium and the graphite.

Segments of the structural unit comprising a compacted mixture of iron and graphite may be made simply and economically as small pressings of the mixture of iron, graphite and, when present, copper.

Each segment may have a length of from 5 to 20mm so as to provide a structural unit having a total length of about 20 - 100mm, especially 60-80mm, typically 70mm. Thus, for treatment of cattle, a typical length of structural unit may be from 15 to 25mm, especially 19 - 21mm, while for treatment of sheep, a typical length may be from 10 to 20mm, especially 14 - 16mm. In each case, a diameter of from 15 to 25mm, especially 18 - 20mm, is suitable for both cattle and sheep. The segment length is determined by the required pulse interval, which intervals need not always be equal. Thus, it is possible to include, within a given release device, segments of different respective lengths.

Preferably, the pressure under which the iron-graphite member is compacted is at least about 5 tonnes/cm² and more preferably at least about 6 tonnes/cm². An upper limit of pressure is about 10 tonne/cm². Suitably, the pressure is in the range of about 6 tonne/cm² to about 9 tonne/cm². A pressure of about 7 - 8 tonne/cm² is particularly preferred.

The term 'non-degradable' as used herein with reference to the material of structural units and segments thereof means that the material in question does not degrade in rumen juices on a timescale relevant to the administration of the active agents described herein. The non-degradable materials are therefore preferably resistant to degradation by chemical, biological or physical processes that might be experienced when administered to a ruminant.

### Brief Description of the Drawings

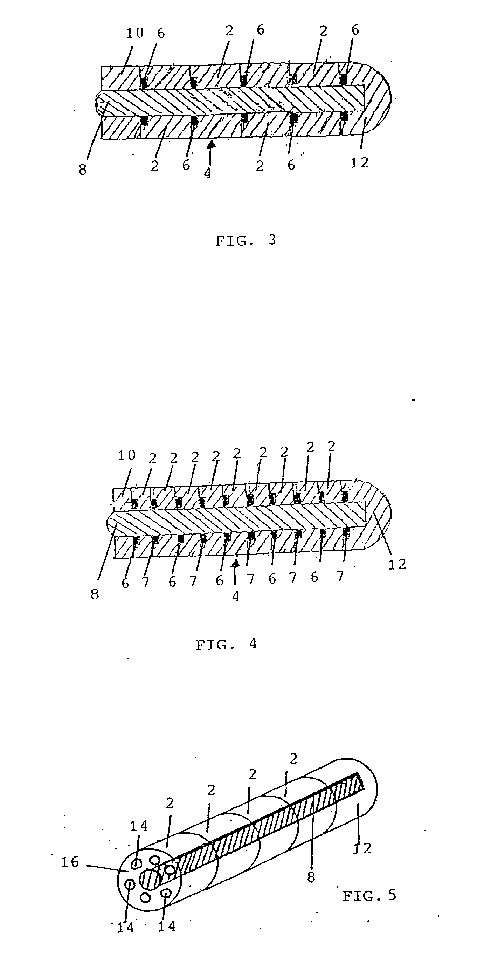

Embodiments of the invention will now be described, by way of example only, with reference to the accompanying drawings of such embodiments and, by way of comparison, drawings of conventional boli. In the drawings (where Figs. 1 to 4 are all in longitudinal section, while Fig. 5 is a schematic illustration):
Figure 1 shows a first conventional bolus in accordance with EP-A-0164927, as has already been discussed;
Figure 2 shows a second conventional bolus in accordance with EP-A-0243111, again as previously discussed;
Figure 3 shows a release device, being a first embodiment of the present invention;
Figure 4 shows a second release device, being a second embodiment of the present invention; and
Figure 5 shows a third release device, being a third embodiment of the present invention.

### Detailed Description of Embodiments and Experiments

The simplicity of bolus designs embodying this invention may be seen from the longitudinal section of the bolus shown in Figure 3. Here, structural elements, in the form of compacts of iron, graphite and optionally copper, including respective segments [2] of a structural unit, generally indicated by arrow [4], carrying active tablets [6], are supported on a magnesium alloy rod [8]. A blank structural element [10] provides an initial delay after administration and before an active tablet [6] is released, while a structural element [12], in addition to carrying a tablet, is profiled so as to provide a covering element. The release mechanism is similar to that of the bolus shown in Figure 1 in that dissolution and shortening of the rod by galvanic action in the rumen fluid results in the progressive discard, firstly of the blank structural element [10], and then of the segments [2] and release of the active tablets [6] containing, for example, ivermectin. Typically, the bolus of Figure 3 has a length of 70mm and a diameter of 19mm.

Figure 4 illustrates a further and more complex device suitable for the delivery of multi actives, particularly for sheep, where resistance problems to the benzimidazoles and ivermectin are being increasingly encountered. Here the segments [2], of iron, graphite and optionally copper hereinafter referred to as "iron-copper-graphite segments [2]" are approximately half the length of those shown in the bolus of Figure 3. A tablet is hence released at approximately 10 day intervals. The bolus contains for example three actives, benzimidazole and ivermectin in tablets [6] and levamisole in tablets [7]. Trace elements such as cobalt and selenium in the form of soluble compounds may also be incorporated in the active tablets. Each drug is released at 20 day intervals and the active content of the bolus is twice that of the bolus illustrated in Figure 3. This is gained at the expense of a more complex assembly operation but little difference in segment cost due to the high rate of production. Again, typically, the bolus of Figure 4 has a length of 70mm and a diameter of 19mm.

Figure 5 is a schematic illustration of yet another release device embodying the invention in which, in place of the single recess for a ring shaped tablet dosage [6] as provided in the device of Figures 3 and 4, here, a plurality of recesses [14], each spaced from the central core, are provided in each iron-copper-graphite segment [2] for respective disc-shaped tablets. The bolus of Figure 5 has a closed end provided by a covering element [12] and an open end [16], so that on administration to an animal of a tablet dosage is immediately released into the animal.

The economy and simplicity of the devices shown in Figures 3 to 5 is achieved by the use of a novel iron-graphite segment which has the following advantages of ease and speed of manufacture and economy of materials, resulting in simplicity of bolus assembly and flexibility of bolus design.

### Manufacture of Structural Unit Segments.

A uniform blend of fine, e.g. 300 mesh [BSS 410], iron, graphite and copper powders is readily prepared in any of a variety of industrial and pharmaceutical mixers. In general, the powder mix has little tendency to segregate and can be pressed into segments at high speed on a rotary tablet machine. Very high rates of output can be achieved e.g. 70,000 segments per hour. In contrast a typical 50 tonne capacity injection moulding machine will produce about 600 segments of plastics material similar to those shown in Figure 1 per hour. For a given bolus output, which may amount to several million per year, substantial savings may be made in production and capital investment costs. Energy savings are particularly significant.

In cases where the strengths of segments produced on rotary machines are inadequate, due either to limitation of the loads that may be applied by particular presses or the ability of the tooling used to withstand the stresses involved, then recourse may be had to heat treatment of the segments. Standard sintering procedures such as are commonly used with iron powder pressings may be applied to the iron-copper-graphite segments of this invention. Substantial improvements in segment strengths may be achieved. However, this is gained at the expense of increased permeability to water penetration, loss of lubricity and increased cost. Heat treatment in steam atmospheres is also applicable at temperatures in excess of about 400°C.

### Economy of Materials.

The segments, shown in Figure 1, of plastics material are made from high grade unplasticised polyvinyl chloride, a material with good physical and chemical properties for bolus use. However, the yield of segments is typically less than 70% of the material processed. This is due to degradation of the plastics material at the moulding temperature and inability to recycle process scrap. In contrast the yield of segments from iron-graphite powder can exceed 95%. Although iron-graphite is more expensive on a volume basis than polyvinyl chloride its high density means that the weight [j] shown in Figure 1 of the conventional bolus can be dispensed with, offsetting the volume price advantage of the plastics material. Similarly the washers [e] sealing the junctions between adjacent segments are not required for iron-graphite due to the water repellant nature and high degree of dimensional accuracy and quality of surface finish of the latter.

### Simplicity of Bolus Assembly.

Reductions in the number and types of component lead to an automated assembly process using less complex and fewer machines with production time and capital cost savings.

Thus, a preferred bolus in accordance with the invention, in which the structural unit comprises a plurality of structural elements each having a central aperture and including segments carrying tablets of active agent composition, can be manufactured simply by providing the tablets in respective recesses in axial end faces of the segments, stacking the structural elements one above the other in axial alignment in a suitable housing so as to assemble a structural element having a central aperture and pressing a core of degradable material into the central aperture of the structural unit with an interference fit to obtain a bolus.

### Flexibility of Bolus Design.

The interval between release of doses of active agent, e.g. in tablet form, can be readily changed by altering the length of the iron-copper-graphite segments. Additional methods including varying the characteristics of the degradable member, for example, where this is a magnesium alloy support rod, adjusting the diameter of the rod and changing the alloy composition. Data contained in WO-A-98/56347 illustrates how the alloy composition may be changed.

### Active Tablet Manufacture

This follows standard pharmaceutical practice. A variety of binders, lubricants and bulking agents including polyvinylpyrrolidone, starch, magnesium stearate and lactose may be included.

### Graphite Content

The concentration of graphite in an iron-graphite segment of a structural unit affects the properties of the segment material. Graphite provides lubricity to the non-degradable component and also helps prevent moisture penetration of the segments by the rumen contents.

Thus, very low graphite concentrations, especially less than about 2wt%, may not be desirable because concentrations below this level may provide insufficient lubricity and protection against ingress of moisture.

Similarly, high graphite concentrations are less desirable because high concentrations, e.g. more than about 7wt%, reduce the strength of the iron-graphite segment. High graphite concentrations may also reduce the density of the device to an extent where the density is no longer sufficient to prevent regurgitation of the device without the addition of supplementary weights.

It is not desirable for the non-degradable member to be made entirely from iron powder due to problems with ingress of water caused by porosity.

The porosity of the non-degradable member can be reduced by increasing the pressure applied to the iron-graphite mixture when it is compacted. Increased pressure also increases the strength of the non-degradable member. Generally, the upper limit for the pressure that can be applied to the iron-graphite mixture, and hence the strength of the non-degradable member, is governed by the maximum capacity of the rotary tablet press to apply force (pressure) to the mixture. Typically the maximum force that a conventional rotary tablet press can apply is about 20 tonnes. Higher forces may be applied, and hence potentially higher component strengths achieved, if single stroke presses are used. However, productivity is then much reduced.

Table 1 gives the results of a series of transverse rupture tests carried out on solid, circular section, iron-graphite discs having varying concentrations of graphite. Such tests, carried out on a simpler, solid construction, give more reliable results than a test carried out on a more complex, annular construction of segment in accordance with a preferred embodiment of the invention. Moreover, they still allow the behaviour of a corresponding segment in dependence upon graphite concentration to be predicted. The discs were formed by mixing 300 mesh iron and graphite powders as discussed above, and then pressing them. The disc size was 26 mm diameter x 6 mm thick. A preferred iron-graphite composition for compacting into a structural element in accordance with the invention can preferably withstand a transverse rupture load of at least, and more preferably in excess of, 200kg when pressed into the abovementioned form of a 26 x 6 mm solid disc.

**Table 1**

| Graphite wt % | Pressure tonne/cm² | Segment density g/cm³ | Rupture Load kg |
|---|---|---|---|
| 0 | 3.8 | 6.3 | >245* |
| 1 | 3.8 | 6.2 | >245 |
| 2 | 3.8 | 6.2 | >245 |
| 3 | 3.8 | 6.2 | 243 |
| 4 | 3.8 | 6.2 | 210 |
| 5 | 3.8 | 6.1 | 175 |
| 7 | 3.8 | 5.8 | 179 |
| 10 | 3.8 | 5.6 | 77 |
| 15 | 3.8 | 5.2 | 95 |
| 100 | 3.8 | 2.1 | 12 |
| 0 | 6.6 | 6.5 | >245 |
| 1 | 6.6 | 6.5 | >245 |
| 2 | 6.6 | 6.5 | >245 |
| 5 | 6.6 | 6.5 | 240 |
| 7 | 6.6 | 6.4 | 216 |
| 10 | 6.6 | 6.5 | 144 |
| 100 | 6.6 | 2.0 | 36 |

| | | | |
|---|---|---|---|
| * Maximum capacity of testing machine | | | |

As can be seen from the tabulated results, the relative proportions of iron and graphite in the powder mix can be adjusted to provide the non-degradable segments with adequate strength to survive the bolus assembly stresses without failure. In embodiments having a magnesium alloy support rod, insertion of this through a central aperture in the segments can impose stresses on the iron-graphite segments which can cause them to crack if the interference fit between the rod and the segments is too great for the strength of the compacted iron-graphite material.

In manufacture, the degree of variation of the interference fit is governed primarily by the tolerance on the rod diameter. With good production practice this can preferably be limited to e.g. +/- 0.01 mm on a typical 7 mm diameter rod.

Tests have indicated that segments of the type shown in Fig. 3 should be pressed at loads in excess of 6 tonnes/cm², based on projected area, to ensure freedom from cracking when rods are inserted with a low tolerance interference fit.

The transverse rupture tests listed in Table 1 show that a reduction of segment strength may occur where the graphite concentration exceeds about 7wt%.

Copper Content of Iron-Graphite-Copper Segment Pressings.

Copper powder is a useful component of segments according to the present invention. It significantly increases the strength of the compacts. Table 2 gives the results of transverse rupture tests carried out, in the manner described with reference to Table 1, on solid 26mm diameter x 4.7 mm thick iron-graphite powder compacts containing 5 and 7wt% of graphite with an addition of 3wt% copper. To bring the recorded strengths within the range of the tablet testing machine the thickness of the tablets was reduced from the 6mm employed with the tests of Table 1 to 4.7mm. The test results illustrate the significant strengthening effect of a 3wt% copper powder addition to the iron-graphite tablets.

**Table 2**

| Graphite wt% | Pressure tonne/cm² | Copper wt% | Rupture Load Kg |
|---|---|---|---|
| 5 | 3.8 | 0 | 136 |
| 5 | 3.8 | 3 | 150 |
| 7 | 6.6 | 0 | 171 |
| 7 | 6.6 | 3 | 188 |

## Claims

1. A structural element suitable for use, as a segment of a structural unit comprising an assembly of the structural elements, in the manufacture of a release device for intraruminal administration, to a ruminant animal, of an active agent housed in the structural unit, which structural element is of a compacted material, which material comprises a mixture of powdered iron, graphite and copper, the graphite being present in the mixture in an amount of from 2 to 7wt%, the copper in an amount of up to 5wt% and the iron in an amount of from 88 to 98wt%, by weight of the total weight of iron, copper and graphite, and which structural element has, in at least one end face thereof a recess, adapted to receive a dosage form of the active agent, and has, passing through the structural element, a central aperture adapted to receive a core of anodic material.

2. A structural element according to claim 1, wherein the compacted material consists of a mixture of powdered iron, copper and graphite.

3. A structural element according to claim 1 or claim 2, wherein the compacted material contains at least 1 wt% copper, based on the total weight of the compacted material

4. A structural element according to any one of the preceding claims, wherein the amount of copper in the structural element is from 1 to 4wt% by weight of total material.

5. A structural element according to any one of the preceding claims, having in at least one end face thereof a plurality of recesses, each adapted to receive a respective said dosage form.

6. A structural element according to any one of the preceding claims, having at least one recess in each of opposite end faces thereof, whereby, in the said assembly of structural elements, the or each recess in an end face of a structural element is in register with a corresponding recess in an adjacent end face of an adjacent structural element, respective corresponding recesses thereby cooperating with one another to house a single dosage form.

7. A structural element according to any one of the preceding claims, having a radial cross section which is circular.

8. A structural element according to claim 7, which is cylindrical.

9. A structural element according to claim 7 or claim 8, wherein the central aperture therein is a cylindrical aperture, coaxial with the structural element and adapted to receive, with an interference fit, a cylindrical core of anodic material.

10. A release device for intraruminal administration of an active agent to a ruminant animal, the device having a degradable member, a non-degradable structural unit and an active agent composition carried by the non-degradable structural unit, which structural unit is of a compacted material, which material comprises a mixture of powdered iron, graphite and copper, the graphite being present in the mixture in an amount of from 2 to 7wt%, the copper in an amount of up to 5wt%, and the iron in an amount of from 88 to 98wt%, by weight of the total weight of iron, copper and graphite, whereby the compacted material is capable of enhancing the density of the release device and capable of promoting galvanic corrosion, by rumen juices, of the degradable member, in which release device the degradable member has a central core surrounded by the structural unit, whereby a substantial proportion of the peripheral surface of the central core is protected, by the structural unit, from corrosion by rumen juices, and the structural unit comprises a plurality of segments in face to face relation with one another, each segment having a central aperture through which the central core passes and a plurality of adjacent said segments each having at least one recess receiving a dosage form of the active agent composition, at least one longitudinal end of the central core being open to corrosion, whereby progressive corrosion of the central core causes release from the device of successive segments of the structural unit and of the dosage form of the active agent associated with the said segments.

11. A release device according to claim 10, wherein the compacted material consists of a mixture of iron, copper and graphite.

12. A release device according to claim 10 or claim 11, wherein the compacted material contains at least 1wt% copper, based on the total weight of the compacted material.

13. A release device according to any one of claims 10 to 12, wherein the amount of copper in the structural element is from 1 to 4wt% by weight of total material.

14. A release device according to any one of claims 10 to 13, wherein the degradable member comprises a magnesium alloy.

15. A release device according to any one of claims 10 to 14, wherein each of a plurality of adjacent segments in face to face relation with one another has one axial end face having at least one said recess in face to face relation with an axial end face of an adjacent segment free from a said recess.

16. A release device according to any one of claims 10 to 14, wherein each of a plurality of adjacent segments in face to face contact with one another has at least one recess in each of opposite end faces thereof, the or each recess in an axial end face of a segment being in register with a corresponding recess in an adjacent axial end face of an adjacent segment, whereby respective corresponding recesses cooperate with one another to house a dosage form.

17. A release device according to any one of claims 10 to 16, wherein segments in face to face relation with each other have, in at least one axial end thereof, a plurality of recesses each capable of receiving a dosage form of the active agent composition.

18. A release device according to any one of claims 10 to 17, wherein the structural unit additionally comprises a covering element adapted to cover a longitudinal end of the central core opposite the said longitudinal end thereof to prevent exposure thereof.

19. A release device according to any one of claims 10 to 18, wherein the structural unit comprises a plurality of annular section segments having respective central apertures together defining a central aperture of the structural unit and the degradable member is a solid cylindrical rod making an interference fit within the central aperture of the structural unit.

20. A sustained release bolus comprising
a structural unit comprising a plurality of annular segments in face to face relation with one another and having respective central apertures together defining a central cylindrical aperture of the structural unit,
a central cylindrical core of a magnesium alloy making an interference fit within the central cylindrical aperture of the structural unit;
the structural unit including segments having, in at least one axial end face thereof, at least one recess; and
a dosage form of a medicament housed in each respective recess of the segments;
each annular segment comprising a compacted material which material comprises a mixture of powdered iron, graphite and copper, the graphite being present in the mixture in an amount of from 2 to 7wt%, the copper in an amount of up to 5wt% and the iron in an amount of 88 to 98wt%, by weight of the total weight of iron, copper and graphite, which compacted material is capable of enhancing the density of the bolus and promoting galvanic corrosion of the magnesium alloy core; and
at least one axial end region of the core being exposed to allow the said galvanic corrosion thereof.

21. A sustained release bolus according to claim 20, wherein the amount of copper in the annular segment is from 1 to 4wt% by weight of total material.

22. Use, as a non-degradable structural element of a structural unit comprising an assembly of the structural elements in the manufacture of a release device for intraruminal administration, to a ruminant animal, of an active agent housed in the structural unit, which non-degradable structural element is made from a compacted material comprising a mixture of powdered iron, graphite and copper, the graphite being present in the mixture in an amount of from 2 to 7wt%, the copper in an amount of up to 5wt% and the iron in an amount of 88 to 98wt%, by weight of the total weight of iron, copper and graphite, which compacted material is capable of enhancing the density and capable of promoting galvanic corrosion, by rumen juices, of a degradable member of the release device, and which structural element has, in at least one end face thereof a recess, adapted to receive a dosage form of the active agent, and has, passing through the structural element, a central aperture adapted to receive a core of anodic material.

23. Use according to claim 22, wherein the compacted material consists of a mixture of iron, graphite and copper.

24. Use according to claim 22 or claim 23, wherein the compacted material contains at least 1wt% copper, based on the total weight of the compacted material.

25. Use according to any one of claims 22 to 24, wherein the amount of copper in the structural element is from 1 to 4wt% by weight of total material.

## Patentansprüche

1. Strukturelement, das zur Verwendung als Segment einer Struktureinheit, die eine Anordnung der Strukturelemente umfasst, bei der Herstellung einer Freisetzungsvorrichtung zur intraruminalen Verabreichung eines in der Struktureinheit untergebrachten Wirkstoffs an ein wiederkäuendes Tier geeignet ist, wobei das Strukturelement aus einem kompaktierten Material besteht, das ein Gemisch aus Eisenpulver, Graphit und Kupfer umfasst, wobei der Graphit in dem Gemisch in einer Menge von 2 bis 7 Gew.-%, das Kupfer in einer Menge von bis zu 5 Gew.-% und das Eisen in einer Menge von 88 bis 98 Gew.-%, jeweils bezogen auf das Gesamtgewicht von Eisen, Kupfer und Graphit, vorhanden ist, wobei das Strukturelement an zumindest einer seiner Stirnflächen eine Aussparung aufweist, die zur Aufnahme einer Dosierungsform des Wirkstoffs angepasst ist, und eine durch das Strukturelement verlaufende zentrale Öffnung aufweist, die zur Aufnahme eines Kerns aus anodischem Material angepasst ist.

2. Strukturelement nach Anspruch 1, worin das kompaktierte Material aus einem Gemisch aus Eisenpulver, Kupfer und Graphit besteht.

3. Strukturelement nach Anspruch 1 oder Anspruch 2, worin das kompaktierte Material zumindest 1 Gew.-% Kupfer, bezogen auf das Gesamtgewicht des kompaktierten Materials, enthält.

4. Strukturelement nach einem der vorangegangenen Ansprüche, worin die Menge an Kupfer in dem Strukturelement 1 bis 4 Gew.-% des Gesamtmaterials beträgt.

5. Strukturelement nach einem der vorangegangenen Ansprüche, das an zumindest einer seiner Stirnflächen eine Vielzahl von Aussparungen aufweist, die jeweils zur Aufnahme einer entsprechenden Dosierungsform angepasst sind.

6. Strukturelement nach einem der vorangegangenen Ansprüche, das zumindest eine Aussparung an jeder seiner gegenüberliegenden Stirnflächen aufweist, wodurch in der Anordnung von Strukturelementen die oder jede Aussparung an einer Stirnfläche eines Strukturelements deckungsgleich mit einer entsprechenden Aussparung an einer benachbarten Stirnfläche eines benachbarten Strukturelements ist, wodurch jeweils einander entsprechende Aussparungen miteinander darin zusammenwirken, eine einzelne Dosierungsform darin unterzubringen.

7. Strukturelement nach einem der vorangegangenen Ansprüche, das einen radialen Querschnitt aufweist, der kreisförmig ist.

8. Strukturelement nach Anspruch 7, das zylindrisch ist.

9. Strukturelement nach Anspruch 7 oder Anspruch 8, worin die zentrale Öffnung darin eine mit dem Strukturelement koaxiale zylindrische Öffnung ist, die zur Aufnahme eines zylindrischen Kerns aus anodischem Material mittels Presspassung angepasst ist.

10. Freisetzungsvorrichtung zur intraruminalen Verabreichung eines Wirkstoffs an ein wiederkäuendes Tier, wobei die Vorrichtung ein abbaubares Element, eine nicht abbaubare Struktureinheit und eine Wirkstoffzusammensetzung aufweist, die von der nicht abbaubaren Struktureinheit getragen wird, wobei diese Struktureinheit aus einem kompaktierten Material besteht, das ein Gemisch aus Eisenpulver, Graphit und Kupfer umfasst, wobei der Graphit in dem Gemisch in einer Menge von 2 bis 7 Gew.-%, das Kupfer in einer Menge von bis zu 5 Gew.-% und das Eisen in einer Menge von 88 bis 98 Gew.-%, jeweils bezogen auf das Gesamtgewicht von Eisen, Kupfer und Graphit, vorhanden ist, wodurch das kompaktierte Material in der Lage ist, die Dichte der Freisetzungsvorrichtung zu erhöhen, und in der Lage ist, galvanische Korrosion des abbaubaren Elements durch Pansensäfte zu fördern, wobei in der Freisetzungsvorrichtung das abbaubare Element einen zentralen Kern aufweist, der von der Struktureinheit umgeben ist, wodurch ein wesentlicher Anteil der peripheren Oberfläche des zentralen Kerns durch die Struktureinheit vor Korrosion durch Pansensäfte geschützt ist, und die Struktureinheit eine Vielzahl von Segmenten in gegenüberliegender Beziehung zueinander umfasst, wobei jedes Segment eine zentrale Öffnung aufweist, durch die der zentrale Kern verläuft, und eine Vielzahl der Segmente, die zueinander benachbart sind, jeweils zumindest eine Aussparung zur Aufnahme einer Dosierungsform der Wirkstoffzusammensetzung aufweisen, wobei zumindest ein Längsende des zentralen Kerns gegenüber Korrosion freiliegt, wodurch fortschreitende Korrosion des zentralen Kerns die Freisetzung aufeinander folgender Segmente der Struktureinheit und der mit den Segmenten verbundenen Dosierungsform des Wirkstoffs aus der Vorrichtung verursacht.

11. Freisetzungsvorrichtung nach Anspruch 10, worin das kompaktierte Material aus einem Gemisch aus Eisen, Kupfer und Graphit besteht.

12. Freisetzungsvorrichtung nach Anspruch 10 oder Anspruch 11, worin das kompaktierte Material zumindest 1 Gew.-% Kupfer, bezogen auf das Gesamtgewicht des kompaktierten Materials, enthält.

13. Freisetzungsvorrichtung nach einem der Ansprüche 10 bis 12, worin die Menge an Kupfer in dem Strukturelement 1 bis 4 Gew.-% des Gesamtmaterials ausmacht.

14. Freisetzungsvorrichtung nach einem der Ansprüche 10 bis 13, worin das abbaubare Element eine Magnesiumlegierung umfasst.

15. Freisetzungsvorrichtung nach einem der Ansprüche 10 bis 14, worin jedes einer Vielzahl von benachbarten Segmenten in gegenüberliegender Beziehung zueinander eine axiale Stirnfläche mit zumindest einer besagten Aussparung in gegenüberliegender Beziehung zu einer axialen Stirnfläche eines benachbarten Segments ohne eine solche Aussparung aufweist.

16. Freisetzungsvorrichtung nach einem der Ansprüche 10 bis 14, worin jedes einer Vielzahl von benachbarten Segmenten in gegenüberliegendem Kontakt miteinander zumindest eine Aussparung in jeder seiner gegenüberliegenden Stirnflächen aufweist, wobei die oder jede Aussparung in einer axialen Stirnfläche eines Segments deckungsgleich mit einer entsprechenden Aussparung an einer benachbarten axialen Stirnfläche eines benachbarten Segments ist, wodurch jeweils einander entsprechende Aussparungen miteinander darin zusammenwirken, eine einzelne Dosierungsform unterzubringen.

17. Freisetzungsvorrichtung nach einem der Ansprüche 10 bis 16, worin Segmente in gegenüberliegender Beziehung zueinander an zumindest einem ihrer axialen Enden eine Vielzahl von Aussparungen aufweisen, die jeweils in der Lage sind, eine Dosierungsform der Wirkstoffzusammensetzung aufzunehmen.

18. Freisetzungsvorrichtung nach einem der Ansprüche 10 bis 17, worin die Struktureinheit zusätzlich ein Deckelement umfasst, das zum Abdecken eines Längsendes des zentralen Kerns gegenüber dessen besagten Längsendes angepasst ist, um dessen Freilegung zu verhindern.

19. Freisetzungsvorrichtung nach einem der Ansprüche 10 bis 18, worin die Struktureinheit eine Vielzahl von ringförmigen Segmenten jeweils mit entsprechenden zentralen Öffnungen umfasst, die zusammen eine zentrale Öffnung der Struktureinheit definieren, und das abbaubare Element ein fester, zylindrischer Stab ist, der eine Presspassung innerhalb der zentralen Öffnung der Struktureinheit herstellt.

20. Bolus zur verzögerten Freisetzung, der Folgendes umfasst:
eine Struktureinheit, die eine Vielzahl von ringförmigen Segmenten in gegenüberliegender Beziehung zueinander umfasst, die jeweils entsprechende zentrale Öffnungen aufweisen, die zusammen eine zentrale zylindrische Öffnung der Struktureinheit definieren,
einen zentralen zylindrischen Kern aus einer Magnesiumlegierung, der eine Presspassung innerhalb der zentralen zylindrischen Öffnung der Struktureinheit herstellt;
wobei die Struktureinheit Segmente umfasst, die an zumindest einer ihrer axialen Stirnflächen zumindest eine Aussparung aufweisen; und
eine Dosierungsform eines Medikaments, das jeweils in den entsprechenden Aussparungen der Segmente untergebracht ist;
wobei jedes ringförmige Segment ein kompaktiertes Material umfasst, wobei das Material ein Gemisch aus Eisenpulver, Graphit und Kupfer umfasst, wobei der Graphit in dem Gemisch in der Menge von 2 bis 7 Gew.-%, das Kupfer in einer Menge von bis zu 5 Gew.-% und das Eisen in einer Menge von 88 bis 98 Gew.-%, jeweils bezogen auf das Gesamtgewicht von Eisen, Kupfer und Graphit, vorhanden ist, wobei das kompaktierte Material in der Lage ist, die Dichte des Bolus zu erhöhen und galvanische Korrosion des Magnesiumlegierungskerns zu fördern; und
wobei zumindest ein axialer Endbereich des Kerns freiliegt, um dessen galvanische Korrosion zu ermöglichen.

21. Bolus zur verzögerten Freisetzung nach Anspruch 20, worin die Menge an Kupfer in dem ringförmigen Segment 1 bis 4 Gew.-% des Gesamtmaterials ausmacht.

22. Verwendung einer Struktureinheit als nicht abbaubares Strukturelement, die eine Anordnung der Strukturelemente umfasst, zur Herstellung einer Freisetzungsvorrichtung zur intraruminalen Verabreichung eines in der Struktureinheit untergebrachten Wirkstoffs an ein wiederkäuendes Tier, wobei das nicht abbaubare Strukturelement aus einem kompaktierten Material besteht, das ein Gemisch aus Eisenpulver, Graphit und Kupfer umfasst, wobei der Graphit in dem Gemisch in der Menge von 2 bis 7 Gew.-%, das Kupfer in einer Menge von bis zu 5 Gew.-% und das Eisen in einer Menge von 88 bis 98 Gew.-%, jeweils bezogen auf das Gesamtgewicht von Eisen, Kupfer und Graphit, vorhanden ist, wobei das kompaktierte Material in der Lage ist, Dichte des Bolus zu erhöhen, und in der Lage ist, galvanische Korrosion eines abbaubaren Elements der Freisetzungsvorrichtung durch Pansensäfte zu fördern, und wobei das Strukturelement in zumindest einer seiner Stirnflächen eine Aussparung aufweist, die zur Aufnahme einer Dosierungsform des Wirkstoffs angepasst ist, und eine durch das Strukturelement verlaufende zentrale Öffnung aufweist, die zur Aufnahme eines Kerns aus anodischem Material angepasst ist.

23. Verwendung nach Anspruch 22, worin das kompaktierte Material aus einem Gemisch aus Eisen, Graphit und Kupfer besteht.

24. Verwendung nach Anspruch 22 oder Anspruch 23, worin das kompaktierte Material zumindest 1 Gew.-% Kupfer, bezogen auf das Gesamtgewicht des kompaktierten Materials, enthält.

25. Verwendung nach einem der Ansprüche 22 bis 24, worin die Menge an Kupfer in dem Strukturelement 1 bis 4 Gew.-% des Gesamtmaterials ausmacht.

## Revendications

1. Elément structurel apte à être utilisé, en tant que segment d'une unité structurelle comprenant un ensemble d'éléments structurels, dans la fabrication d'un dispositif à libération pour l'administration intra-ruminale, à un animal ruminant, d'un agent actif logé dans l'unité structurelle, ledit élément structurel est en un matériau compacté, ledit matériau comprend un mélange de fer, graphite et cuivre en poudre, le graphite étant présent dans le mélange en une quantité de 2 à 7 % en poids, le cuivre en une quantité jusqu'à 5 % en poids et le fer en une quantité de 88 à 98 % en poids, par poids tu poids total de fer, cuivre et graphite, et ledit élément structurel présente, dans au moins une face d'extrémité de celui-ci, un évidemment apte à recevoir une forme de dosage de l'agent actif, et présente, passant à travers l'élément structurel, une ouverture centrale apte à recevoir un noyau en matériau anodique.

2. Elément structurel selon la revendication 1, dans lequel le matériau compacté consiste en un mélange de fer, cuivre et graphite en poudre.

3. Elément structurel selon la revendication 1 ou la revendication 2, dans lequel le matériau compacté contient au moins 1 % en poids de cuivre, basé sur le poids total du matériau compacté.

4. Elément structurel selon l'une quelconque des revendications précédentes, dans lequel la quantité de cuivre dans l'élément structurel est de 1 à 4 % en poids de la totalité du matériau.

5. Elément structurel selon l'une quelconque des revendications précédentes, ayant dans au moins une face d'extrémité de celui-ci une pluralité d'évidements, chacun apte à recevoir une forme de dosage respective précitée.

6. Elément structurel selon l'une quelconque des revendications précédentes, ayant au moins un évidement dans chacun de ses faces d'extrémité opposées, moyennant quoi, dans ledit ensemble d'éléments structurels, le ou chaque évidement dans une face d'extrémité d'un élément structurel coïncide avec un évidement correspondant dans une face d'extrémité adjacente d'un élément structurel adjacent, des évidements respectifs correspondants coopérant ainsi les uns avec les autres pour renfermer une seule forme de dosage.

7. Elément structurel selon l'une quelconque des revendications précédentes, ayant une section transversale radiale qui est circulaire.

8. Elément structurel selon la revendication 7, qui est cylindrique.

9. Elément structurel selon la revendication 7 ou la revendication 8, dans lequel l'ouverture centrale dans celui-ci est une ouverture cylindrique, coaxiale avec l'élément structurel et apte à recevoir, avec un ajustement avec serrage, un noyau cylindrique en matériau anodique.

10. Dispositif à libération pour l'administration intra-ruminale d'un agent actif à un animal ruminant, le dispositif ayant un élément dégradable, une unité structurelle non dégradable et une composition d'agent actif supportée par l'unité structurelle non dégradable, ladite unité structurelle est en un matériau compacté, ledit matériau comprend un mélange de fer, graphite et cuivre en poudre, le graphite étant présent dans le mélange en une quantité de 2 à 7 % en poids, le cuivre en une quantité jusqu'à 5 % en poids, et le fer en une quantité de 88 à 98 % en poids, par poids du poids total du fer, du cuivre et du graphite, moyennant quoi le matériau compacté est apte à renforcer la densité du dispositif à libération et est apte à promouvoir la corrosion galvanique, par des jus de rumen, de l'élément dégradable, dans ledit dispositif à libération, l'élément dégradable possède un noyau central entouré par l'unité structurelle, moyennant quoi une proportion substantielle de la surface périphérique du noyau central est protégée, par l'unité structurelle, contre la corrosion par des jus de rumen, et l'unité structurelle comprend une pluralité de segments en une relation de face à face les uns avec les autres, chaque segment ayant une ouverture centrale à travers laquelle le noyau central passe, et une pluralité de segments adjacents ayant chacun au moins un évidement recevant une forme de dosage de la composition d'agent actif, au moins une extrémité longitudinale du noyau central étant ouverte à la corrosion, moyennant quoi une corrosion progressive du noyau central provoque une libération du dispositif de segments successifs de l'unité structurelle et de la forme de dosage de l'agent actif associée auxdits segments.

11. Dispositif à libération selon la revendication 10, dans lequel le matériau compacté consiste en un mélange de fer, cuivre et graphite.

12. Dispositif à libération selon la revendication 10 ou la revendication 11, dans lequel le matériau compacté contient au moins 1 % en poids de cuivre, basé sur le poids total du matériau compacté.

13. Dispositif à libération selon l'une quelconque des revendications 10 à 12, dans lequel la quantité de cuivre dans l'élément structurel est de 1 à 4 % en poids de la totalité du matériau.

14. Dispositif à libération selon l'une quelconque des revendications 10 à 13, dans lequel l'élément dégradable comprend un alliage de magnésium.

15. Dispositif à libération selon l'une quelconque des revendications 10 à 14, dans lequel chacun d'une pluralité de segments adjacents en une relation face à face les uns avec les autres possède une face d'extrémité axiale ayant au moins un évidement précité en une relation face à face avec une face d'extrémité axiale d'un segment adjacent exempt d'un évidement précité.

16. Dispositif à libération selon l'une quelconque des revendications 10 à 14, dans lequel chacun d'une pluralité de segments adjacents en un contact face à face les uns avec les autres possède au moins un évidement dans chacune de ses faces d'extrémité opposées, le ou chaque évidement dans une face d'extrémité axiale d'un segment étant en coïncidence avec un évidement correspondant dans une face d'extrémité axiale adjacente d'un segment adjacent, moyennant quoi des évidements correspondants coopèrent les uns avec les autres pour renfermer une forme de dosage.

17. Dispositif à libération selon l'une quelconque des revendications 10 à 16, dans lequel des segments en une relation face à face les uns avec les autres présentent, dans au moins une extrémité axiale de ceux-ci, une pluralité d'évidements chacun apte à recevoir une forme de dosage de la composition d'agent actif.

18. Dispositif à libération selon l'une quelconque des revendications 10 à 17, dans lequel l'unité structurelle comprend additionnellement un élément de recouvrement apte à couvrir une extrémité longitudinale du noyau central opposé à ladite extrémité longitudinale de celui-ci pour empêcher l'exposition de celui-ci.

19. Dispositif à libération selon l'une quelconque des revendications 10 à 18, dans lequel l'unité structurelle comprend une pluralité de segments de section annulaire ayant des ouvertures centrales respectives définissant ensemble une ouverture centrale de l'unité structurelle, et l'élément dégradable est une tige cylindrique solide établissant un ajustement avec serrage dans l'ouverture centrale de l'unité structurelle.

20. Bolus à libération prolongée comprenant :
une unité structurelle comprenant une pluralité de segments annulaires en une relation face à face les uns avec les autres et ayant des ouvertures centrales respectives définissant ensemble une ouverture cylindrique centrale de l'unité structurelle,
un noyau cylindrique central en un alliage de magnésium établissant un ajustement avec serrage dans l'ouverture cylindrique centrale de l'unité structurelle ;
l'unité structurelle incluant des segments ayant, dans au moins une face d'extrémité axiale de ceux-ci, au moins un évidement ; et
une forme de dosage d'un médicament logée dans chaque évidement respectif des segments ;
chaque segment annulaire comprenant un matériau compacté, ledit matériau comprend un mélange de fer, graphite et cuivre en poudre, le graphite étant présent dans le mélange en une quantité de 2 à 7 % en poids, le cuivre en une quantité jusqu'à 5 % en poids et le fer en une quantité de 88 à 98 % en poids, par poids du poids total du fer, du cuivre et du graphite, ledit matériau compacté est apte à augmenter la densité du bolus et à promouvoir une corrosion galvanique du noyau en alliage de magnésium ; et
au moins une région d'extrémité axiale du noyau étant exposée pour permette ladite corrosion galvanique de celui-ci.

21. Bolus à libération prolongée selon la revendication 20, dans lequel la quantité de cuivre dans le segment annulaire est de 1 à 4 % en poids de la totalité du matériau.

22. Utilisation, comme élément structurel non-dégradable d'une unité structurelle comprenant un ensemble d'éléments structurels dans la fabrication d'un dispositif à libération pour l'administration intra-ruminale, à un animal ruminant, d'un agent actif logé dans l'unité structurelle, ledit élément structurel non-dégradable est réalisé à partir d'un matériau compacté comprenant un mélange de fer, graphite et cuivre en poudre, le graphite étant présent dans le mélange en une quantité de 2 à7 % en poids, le cuivre en une quantité jusqu'à 5 % en poids et le fer en une quantité de 88 à 98 % en poids, par poids du poids total du fer, cuivre et graphite, ledit matériau compacté est apte à augmenter la densité et est apte à promouvoir la corrosion galvanique, par des jus de rumen, d'un élément dégradable du dispositif à libération, et ledit élément structurel possède, dans au moins une face d'extrémité de celui-ci, un évidement apte à recevoir une forme de dosage de l'agent actif, et présente, passant à travers l'élément structurel, une ouverture centrale apte à recevoir un noyau de matériau anodique.

23. Utilisation selon la revendication 22, dans laquelle le matériau compacté consiste en un mélange de fer, graphite et cuivre.

24. Utilisation selon la revendication 22 ou la revendication 23, dans laquelle le matériau compacté contient au moins 1 % en poids de cuivre, basé sur le poids total du matériau compacté.

25. Utilisation selon l'une quelconque des revendications 22 à 24, dans laquelle la quantité de cuivre dans l'élément structurel est de 1 à 4 % en poids de la totalité du matériau.
